# EUROPEAN PATENT APPLICATION

(11) **EP 4 442 643 A1**
(43) Date of publication of application: **09.10.2024**
(21) Application number: 22901265.3
(22) Date of filing: 29.11.2022
(51) Int. Cl.: C01B 32/40, B01D 19/00, B01D 53/04, B01D 53/047, B01D 53/14, B01D 53/22, B01D 53/26, B01D 53/62, B01D 53/82, C10L 3/08

(54) **GAS PRODUCTION DEVICE**

(30) Priority: 30.11.2021 JP 2021194826
(71) Applicant: SEKISUI CHEMICAL CO., LTD., Osaka-shi Osaka 530-8565 (JP)
(72) Inventor: TONO, Tsuyoshi, Tsukuba-shi, Ibaraki 300-4292 (JP); NAKAMURA, Masaki, Tsukuba-shi, Ibaraki 300-4292 (JP); NAKAMA, Yuki, Tsukuba-shi, Ibaraki 300-4292 (JP)
(74) Representative: Alpspitz IP
(86) International application number: PCT/JP2022/043858
(87) International publication number: WO 2023/100833

(57) **Abstract**

[Problem] To provide a gas production device that makes it possible to raise the temperature of a reaction unit by separating a removal unit from a reactor and that can efficiently generate a valuable carbon substance.

[Solution] Provided according to one aspect of the present invention is a gas production device. This gas production device has at least two reactors and a removal unit. The two reactors are connected in series and are configured to be capable of supplying an oxidizing gas that contains carbon dioxide and a reducing gas that contains a reducing substance. Each of the reactors accommodates a metal and/or a metal oxide. The metal and/or metal oxide promotes the reduction of the carbon dioxide or the reaction between the carbon dioxide and the reducing substance, thereby generating a valuable carbon substance. The removal unit is connected between the two reactors and has a phase separation mechanism that carries out phase separation of a gaseous component and a separated component including at least one of a liquid component and a solid component produced through cooling, thereby removing products derived from the gas supplied to the reactors.

## Description

### TECHNICAL FIELD

The present disclosure relates to a gas production apparatus.

### BACKGROUND ART

In recent years, atmospheric concentration of carbon dioxide (CO₂), a greenhouse gas, has been rising. Rising concentrations of carbon dioxide in the atmosphere contribute to global warming. Therefore, it is important to recover carbon dioxide released into the atmosphere. Furthermore, if the recovered carbon dioxide can be converted into valuable carbon substances and reused, a carbon-circulating society can be realized.

As a global measure, the Kyoto Protocol to the United Nations Framework Convention on Climate Change sets, for each industrialized country, a reduction rate for carbon dioxide, a cause of global warming, compared to a 1990 level, and stipulates that the countries jointly achieve the reduction targets within the commitment period.

To meet the reduction targets, as exhaust gas containing carbon dioxide from steelworks, refineries, or thermal power plants are also to be reduced, various technological improvements have been made for reducing carbon dioxide from these industries. One example of such technology is CO₂ capture and storage (CCS). However, this technology has a physical limitation of storage and thus cannot be a fundamental solution.

For example, Patent Document 1 discloses a production apparatus for producing carbon monoxide from carbon dioxide by using cerium oxide containing zirconium.

### PRIOR ART DOCUMENTS

### Patent Document

Patent Document 1: Japanese Patent No. 5858926
Patent Document 2: Japanese Patent Application, Publication No. 2020-23488

### SUMMARY

### Problems to be solved by Invention

However, according to the present inventors' review, Patent Document 1 discloses an invention that specifies a metal oxide for efficiently converting carbon dioxide into carbon monoxide. However, Patent Document only discloses conceptual or general information on production conditions and production apparatus for producing carbon monoxide, as can be seen from the drawings, etc. of Patent Document 1. Therefore, it was found that further technical improvements are needed to produce valuable carbon substances such as carbon monoxide on an industrial scale.

From the perspective of producing carbon monoxide on an industrial scale, it is also important to increase energy efficiency. In the above-described production of carbon monoxide using the metal oxide, produced gas contains unreacted carbon dioxide as well as carbon monoxide. Furthermore, hydrogen is brought into contact with the metal oxide in oxidized state for its regeneration, but gas produced in this stage contains unreacted hydrogen as well as water vapor (water). In consideration of reusing unreacted carbon dioxide and unreacted hydrogen, it is effective to remove carbon monoxide and water, which are reaction products, from the product gas.

For example, Patent Document 2 proposes a configuration of disposing, inside a reactor, a selective permeation membrane (removal section) configured to separate a reaction product from a reaction system.

However, it is difficult to use the configuration of disposing the selective permeation membrane inside the reactor because the selective permeation membrane may deteriorate or may be damaged when the reaction system is at high temperature.

In view of the above circumstances, the present disclosure provides a gas production apparatus capable of efficiently producing a valuable carbon substance by separating a removal section from a reactor and thereby enabling the reaction section to be a higher temperature.

### Means for Solving Problems

According to one aspect of the present disclosure, a gas production apparatus is provided. This gas production apparatus has at least two reactors and a removal section. The two reactors are connected in series and configured to supply oxidizing gas containing carbon dioxide and reducing gas containing reducing substance. Each reactor accommodates at least one of metal and metal oxide. The least one of metal and metal oxide is configured to produce a valuable carbon substance by reducing the carbon dioxide or accelerating reaction of the carbon dioxide with the reducing substance. The removal section is connected between the two reactors and has a phase separating mechanism configured to perform phase separation between a separated component and a gas component, and thereby to remove a product derived from gas supplied to the reactors, the separated component containing at least one of a liquid component and a solid component, which are produced by cooling.

According to such an aspect, a valuable carbon substance can be efficiently produced by using oxidizing gas including carbon dioxide and reducing gas including a reducing substance.

### BRIEF DESCRIPTION OF DRAWINGS

FIG. 1 is a schematic diagram illustrating an overall configuration of a gas production system having a gas production apparatus according to the present disclosure.
FIG. 2 is a schematic diagram illustrating a reaction section according to the first embodiment.
FIG. 3 is a schematic diagram illustrating a configuration of a gas-liquid separating mechanism.
FIG. 4 is a schematic diagram illustrating another configuration of a gas-liquid separating mechanism.
FIG. 5 is a schematic diagram illustrating a reaction section according to the second embodiment.
FIG. 6 is a schematic diagram illustrating a method of switching gas to let pass through the reactor according to the second embodiment.
FIG. 7 is a schematic diagram illustrating another configuration of the gas-liquid separating mechanism.

### DETAILED DESCRIPTION

Hereinafter, a detailed description will be given of preferred embodiments of a gas production apparatus according to the present disclosure illustrated the accompanying drawings.

First, the overall configuration of a gas production system will be described.

### FIRST EMBODIMENT

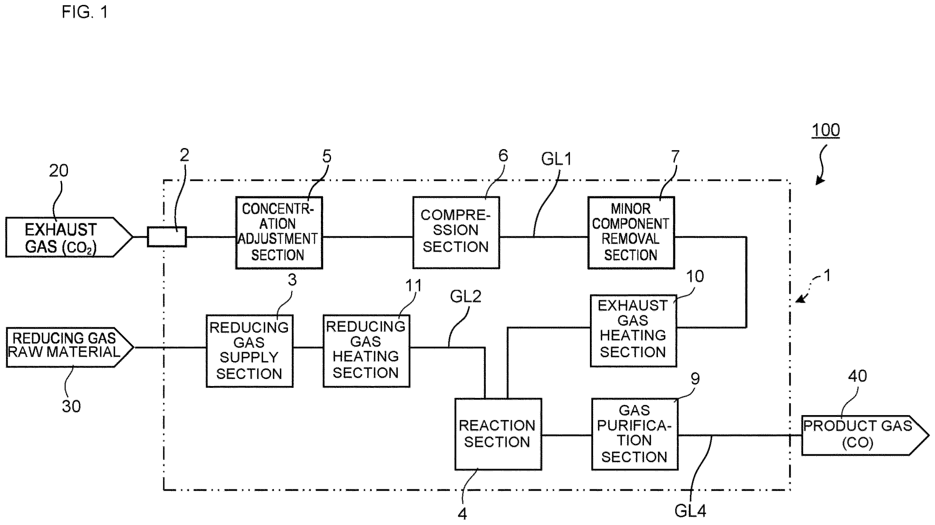

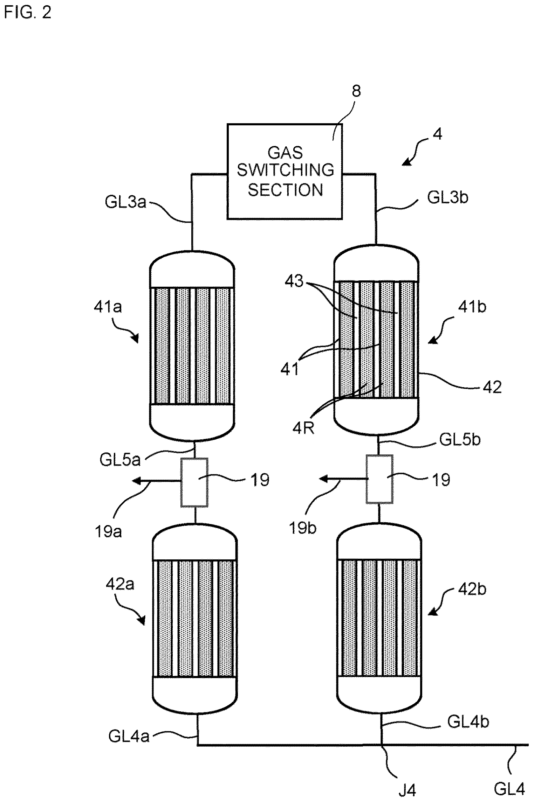

FIG. 1 is a schematic diagram illustrating the overall configuration of the gas production system having the gas production apparatus according to the present disclosure. FIG. 2 is a schematic diagram illustrating a reaction section according to the first embodiment.

The gas production system 100 illustrated in FIG. 1 has a furnace 20 configured to produce exhaust gas (oxidizing gas) containing carbon dioxide and a gas production apparatus 1 connected to the furnace 20 via a connection section 2.

In the present specification, an upstream side of a gas flow direction is also simply referred to as "upstream" and a downstream side of the gas flow direction is also simply referred to as "downstream".

The furnace 20 is not particularly limited, but includes, for example, furnaces attached to steelworks, refineries, or thermal power plants, and may be combustion furnaces, blast furnaces, converters, or the like. The furnace 20 generates (produces) exhaust gas during combustion, melting, refining, or the like of the contents.

In a case of a combustion furnace (incinerator) at a waste incineration plant, the content (waste) includes, for example, plastic waste, food waste, municipal solid waste (MSW), waste tires, biomass waste, household waste (bedding, paper, etc.), construction materials, and the like. The waste may contain one sort alone or two or more sorts of wastes.

In addition to carbon dioxide, the exhaust gas typically contains other gas components such as nitrogen, oxygen, carbon monoxide, water vapor, methane, and the like. The concentration of carbon dioxide in the exhaust gas is not particularly limited, but in view of a production cost of product gas (conversion efficiency into carbon monoxide), the concentration may be 1 vol% (percent by volume) or more, and preferably be 5 vol% or more.

In a case of exhaust gas from a combustion furnace in a waste incineration plant, the exhaust gas contains 5-15 vol% carbon dioxide, 60-70 vol% nitrogen, 5-10 vol% oxygen, and 15-25 vol% water vapor.

Exhaust gas from a blast furnace (blast furnace gas) is gas generated during production of pig iron in the blast furnace and contains 20-30 vol% carbon dioxide, 55-60 vol% nitrogen, 25-30 vol% carbon monoxide, and 1-5 vol% hydrogen.

Exhaust gas from a converter (converter gas) is gas generated during steel production in the converter and contains 15-20 vol% carbon dioxide, 50-60 vol% carbon monoxide, 15-25 vol% nitrogen, and 1-5 vol% hydrogen.

The oxidizing gas is not limited to the exhaust gas, and may be a pure gas containing 100 vol% carbon dioxide.

However, using exhaust gas as oxidizing gas can make it possible to utilize carbon dioxide having been discharged into the atmosphere, which can reduce impact on the environment. Among these, from the perspective of carbon cycling, exhaust gas containing carbon dioxide generated at a steelworks or a refinery may be used.

Blast furnace gas or converter gas may be used as-is as untreated gas discharged from the furnace, or may be used as treated gas after treatment to remove, for example, carbon monoxide, etc. The untreated blast furnace gas and converter gas respectively have gas compositions as described above, and the treated gas has a gas composition similar to the composition described with respect to the exhaust gas from the combustion furnace. In the present specification, any of the above gases (gas before being supplied to the gas production apparatus 1) are referred to as exhaust gas.

The gas production apparatus 1 produces a product gas (synthesis gas) containing carbon monoxide by bringing exhaust gas (oxidizing gas containing carbon dioxide) a reducing agent 4R into contact with each other, the exhaust gas having been discharged from the furnace 20 and supplied via the connection section 2, the reducing agent 4R being configured to reduce carbon dioxide in the exhaust gas.

In the present specification, a description will be given with carbon monoxide as an example of valuable carbon substance. It is to be noted that the valuable carbon substance is not limited to carbon monoxide, and may include, for example, methane, methanol, etc. and may be a single product or a mixture of two or more products. The type of valuable carbon substance produced depends on the type of reducing agent described below.

The gas production apparatus 1 is mainly have the connection section 2, a reducing gas supply section 3, a reaction section 4 having four reactors 41a, 42a, 41b, and 42b (hereinafter, also collectively referred to as "reactors 4ab"), a gas line GL1 connecting the connection section 2 and the reaction section 4, a gas line GL2 connecting the reducing gas supply section 3 and the reaction section 4, and a gas line GL4 connected to the reaction section 4.

In the present embodiment, the connection section 2 functions as an exhaust gas supply section (oxidizing gas supply section) configured to supply exhaust gas to the reaction section 4.

As needed, pumps configured to transfer gas may be disposed at some points along the gas line GL1, the gas line GL2, and the gas line GL4. For example, in a case where a below-described compression section 6 adjusts pressure of the exhaust gas to a relatively low level, disposing a pump allows for smooth transfer of the gas in the gas production apparatus 1.

The gas line GL1 is connected to the connection section 2 at one end. The gas line GL1 is connected at its other end to inlet ports of the reactors 41a and 41b of the reaction section 4 via a gas switching section 8 and respectively via two gas lines GL3a and GL3b as illustrated in FIG. 2.

Such a configuration enables the exhaust gas supplied from the furnace 20 via the connection section 2 to pass through the gas line GL1 and then to be supplied to each of the reactors 41a and 41b.

The gas switching section 8 may include, for example, a branch gas line and a channel opening/closing mechanism such as a valve disposed on this branch gas line.

As illustrated in FIG. 2, each of the reactors 4ab includes a multitubular reactor (fixed layer reactor) having a plurality of tubes 41 each filled with (accommodating) a reducing agent 4R and a housing 42 accommodating the plurality of tubes 41. Such a multitubular reactor can ensure sufficient opportunities for the reducing agent 4R to contact the exhaust gas and reducing gas. This can increase the production efficiency of the product gas.

The reducing agent 4R according to the present embodiment may has a shape (form) of, for example, particles (granules), scales, pellets, or the like. Such a shape of reducing agent 4R can increase the filling efficiency to each tube 41 and can further increase its area to be in contact with gas supplied into the tube 41.

In a case where the reducing agent 4R has a particulate shape, its volume average particle size is not particularly limited, but may be 1-50 mm, and preferably be 3-30 mm. In this case, the area of the contact between the reducing agent 4R and the exhaust gas (carbon dioxide) can be further increased, which can further improve the conversion efficiency from carbon dioxide into carbon monoxide. Similarly, it enables reducing gas including a reducing substance to further efficiently regenerate (reduce) the reducing agent 4R.

The particulate-shaped reducing agent 4R may be shaped particles produced by oscillating granulation so that increase the sphericity of the particulate-shaped reducing agent 4R.

The reducing agent 4R may be carried on a carrier.

The material of the carrier is not particularly limited as long as the material is not easily modified by contact with the exhaust gas (oxidizing gas), by reaction conditions, and the like, and may include, for example, carbon materials (such as graphite, graphene, etc.), carbides such as Mo₂C, oxides such as zeolites, montmorillonites, ZrO₂, TiO₂, V₂O₅, MgO, CeO₂, Al₂O₃, SiO₂, composite oxides including these, and the like.

Among these materials, zeolites, montmorillonites, ZrO₂, TiO₂, V₂O₅, MgO, Al₂O₃, SiO₂, and composite oxides containing these are preferably used as the material of the carrier. The carrier made of such materials may be preferable in not negatively affecting the reaction of the reducing agent 4R and in providing excellent capacity for carrying the reducing agent 4R. The carrier is not involved in the reaction of the reducing agent 4R, but simply supports (holds) the reducing agent 4R.

Such a configuration includes, for example, a configuration where at least part of the surface of the carrier is coated with the reducing agent 4R.

The reducing agent 4R contains at least one of metal and metal oxide (oxygen carrier). The at least one of metal and metal oxide is not particularly limited as long as it can reduce carbon dioxide, but preferably contains at least one selected from metal elements belonging to the third to twelfth groups, more preferably contains at least one selected from metal elements belonging to fourth to twelfth groups, even more preferably contains at least one selected from titanium, vanadium, iron, copper, zinc, nickel, manganese, chromium, cerium, and the like, and particularly preferably is a metal oxide containing iron or a composite metal oxide containing iron. These metal oxides are useful as they exhibit particularly excellent conversion efficiency from carbon dioxide into carbon monoxide.

"Metal" includes single metals each formed by only one of the above-described metal elements and includes alloys each formed by two or more of the above-described metal elements.

In particular, iron oxide, cerium oxide, etc. are suitably used as metal oxides for converting carbon dioxide into carbon monoxide. For example, zirconia, alumina, titania, silica, or the like, which carries or contains at least one of nickel and ruthenium, is suitably used as metal oxide for converting carbon dioxide into methane. For example, zirconia, alumina, silica, or the like, which carries or contains at least one of copper and zinc, is suitably used as metal oxide for converting carbon dioxide into methanol.

In each of the reactors 4ab, the tube (cylindrical shaped body) 41 may be made of the reducing agent 4R (at least one of metal and metal oxide) itself. A block-shaped, lattice (e.g., mesh, honeycomb)-shaped, etc. shaped body may be made of the reducing agent 4R and disposed inside the housing 42. In these cases, the reducing agent 4R as a filler may be omitted or used in combination.

Among these, a preferred configuration may be such that a mesh-like body is made of the reducing agent 4R and disposed inside the housing 42. Such a configuration not only can prevent transmission resistance of the exhaust gas and the reducing gas in each of the reactors 4ab, but also can ensure sufficient opportunities for the reducing agent 4R to contact the exhaust gas and the reducing gas.

The four reactors 41a, 42a, 41b, and 42b are set to have a volume substantially equal to each other, the volume being appropriately set depending on the amount of the exhaust gas to be treated (depending on the size of the furnace 20 and the size of the gas production apparatus 1). The volume of at least one of the four reactors 41a, 42a, 41b, and 42b may be changed depending on the types of the exhaust gas and the reducing gas, the performance of the reducing agent 4R, etc.

On the gas line GL1, disposed are a concentration adjustment section 5, a compression section 6, a minor component removal section 7, and an exhaust gas heating section (oxidizing gas heating section) 10, in order from the connection section 2 side.

The concentration adjustment section 5 is configured to provide adjustment to increase a concentration of carbon dioxide in the exhaust gas (in other words, to concentrate carbon dioxide). The exhaust gas contains unwanted gas components such as oxygen. The concentration adjustment section 5 increasing the concentration of carbon dioxide in the exhaust gas can relatively lower the concentration of the unwanted gas components in the exhaust gas. Thereby, it is possible to prevent or suppress unwanted gas components from negatively affecting the conversion efficiency by reducing agent 4R from carbon dioxide to carbon monoxide.

The concentration adjustment section 5 may function as an oxygen removal apparatus configured to remove oxygen from the exhaust gas. This reduces the amount of oxygen brought into the gas production apparatus 1 (i.e., adjusts the concentration of oxygen in the exhaust gas to low). This allows the gas composition of the exhaust gas to deviate from the explosive range, preventing the exhaust gas from igniting. In the gas production apparatus 1, the oxygen removal apparatus consumes high electrical energy, and thus it is effective to use below-described electricity as a renewable energy.

In this case, the concentration of oxygen in the exhaust gas may be adjusted to less than 1 vol%, preferably adjusted to less than 0. 5 vol%, and more preferably adjusted to less than 0. 1 vol% of the total exhaust gas. This can safely prevent the exhaust gas from igniting.

The oxygen removal apparatus configured to remove oxygen from the exhaust gas may include one or more of the following types of separators: a low-temperature separation (deep cooling) separator, a pressure swing adsorption (PSA) separator, a membrane separation separator, a temperature swing adsorption (TSA) separator, a chemical absorption separator, a chemical adsorption separator, and the like.

The concentration adjustment section 5 may provide adjustment such that carbon dioxide has a higher concentration by adding carbon dioxide to the exhaust gas.

The compression section 6 increases the pressure of the exhaust gas before being supplied to the reactors 4ab. This increases the amount of the exhaust gas that can be treated at once in the reactors 4ab. Thus, it is possible to further improve the conversion efficiently from carbon dioxide into carbon monoxide in the reactors 4ab.

Such a compression section 6 includes, for example, a turbo compressor such as a centrifugal compressor and an axial compressor, positive displacement compressor such as a reciprocating compressor, a diaphragm compressor, a single screw compressor, a twin screw compressor, a scroll compressor, a rotary compressor, a rotary piston type compressor, and a slide vane type compressor, a roots blower usable with low pressure, a centrifugal blower, etc.

Among these, the compression section 6 may be a centrifugal compressor from a viewpoint of easy scaling up of the gas production system 100, and may be a reciprocating compressor from a viewpoint of reduction in the production cost in the gas production system 100.

The pressure of the exhaust gas after passing through the compression section 6 is not particularly limited, but may be 0-1 MPaG, preferably be 0-0.5 MPaG, and more preferably be 0. 01-0.5 MPaG. This can further improve the conversion efficiency from carbon dioxide to carbon monoxide in the reactors 4ab without increasing the pressure resistance of the gas production apparatus 1 beyond necessary.

The minor component removal section 7 removes minor (or minute) components (such as trace amounts of unwanted gas components) contained in the exhaust gas.

Such a minor component removal section 7 may include, for example, at least one of the following treatment units: a gas-liquid separator, a guard reactor, and a scrubber (absorption tower).

In a case where two or more treatment units are used, they may be arranged in any order, but when a gas-liquid separator and a guard reactor are used in combination, the gas-liquid separator may be disposed on the upstream side of the guard reactor. This can further improve the efficiency of removing the minor components from the exhaust gas and can extend the use period (life span) of the guard reactor.

For example, the gas-liquid separator separates, from the exhaust gas, condensed (liquid) water produced during compression of the exhaust gas in compression section 6. In this case, unwanted gas components, etc. remaining in the exhaust gas are also dissolved in the condensed water and removed.

The gas-liquid separator may include, for example, a simple container, a swirling flow separator, a centrifugal separator, a surface tension separator, etc. Among these, the gas-liquid separator may preferably be a simple container as having a simple configuration, being low cost, etc. In this case, at the gas-liquid interface inside the container, the container may have a filter configured to allow passage of gas but blocks passage of liquid.

Furthermore, in this case, a liquid line may be connected to the bottom of the container and a valve may be provided on the liquid line. According to such a configuration, opening the valve can allow the condensed water stored in the container to be discharged from the gas production apparatus 1 through the liquid line.

The liquid line may be connected to a tank 30, described below, so that the discharged condensed water is reused.

For example, configuration may be such that, after the gas-liquid separator removes the condensed water from the exhaust gas, the exhaust gas is supplied to a guard reactor.

Such a guard reactor may contain a substance capable of capturing a component (inactivation component) that is a minor component contained in the exhaust gas and reduces the activity of the reducing agent 4R when contacting the reducing agent 4R.

With such a configuration, since the substance in the guard reactor reacts with (captures) the inactivation component when the exhaust gas passes through the guard reactor, it is possible to prevent or reduce reaching of the inactivation component to the reducing agent 4R in the reactors 4ab and thereby to protect the reducing agent 4R (i.e., to prevent its activity from being reduced). Thus, it is possible to prevent or suppress the negative effect of the inactivation component from extremely reducing the conversion efficiency from carbon dioxide to carbon monoxide by the reducing agent 4R.

Such a substance may include substances having compositions that are contained in the reducing agent 4R and reduce the activity of the reducing agent 4R upon contact with inactivation components, specifically, substances same as or similar to the at least one of metal and metal oxide contained in the reducing agent 4R. In this context, "similar metal oxides" means metal oxides that contain the same metal element as each other but have different compositions from each other or means metal oxides containing metal elements that are different from each other but belong to the same group in the periodic table of elements.

Such an inactivation component may be at least one selected from sulfur, mercury, sulfur compounds, halogen compounds, organic silicone, organic phosphorus, and organic metal compounds, and may preferably be at least one selected from sulfur and sulfur compounds. Removing such an inactivation component in advance allows efficient prevention of rapid reduction in the activity of the reducing agent 4R.

The above substance may be any substance as long as its activity is reduced by the same components as the inactivation components for the reducing agent 4R, and may preferably be metal oxides, such as iron oxide and zinc oxide, as having an excellent ability to capture the above inactivation components.

The guard reactor may be configured such that a mesh element is disposed in a housing and particles of the above substance on the mesh element, such that a honeycomb filter member made of the above substance or a cylindrical or particulate shaped body made of the above substance is disposed in the housing, or the like.

In particular, disposing the guard reactor between the compression section 6 (gas-liquid separator) and the exhaust gas heating section 10 can improve the efficiency of removing the inactivation component while preventing degradation caused by heat from the above substance.

The exhaust gas heating section 10 heats the exhaust gas before being supplied to the reactors 4ab. Preheating the exhaust gas before reaction (before reduction) in the exhaust gas heating section 10 leads to further acceleration in the conversion (reduction) reaction by the reducing agent 4R from carbon dioxide into carbon monoxide in the reactors 4ab.

The exhaust gas heating section 10 may include, for example, an electric heater and a heat exchanger (economizer).

The heat exchanger is formed by bending part of piping of the gas line GL4 configured to discharge gas (mixed gas) after passing through the reactors 4ab and bringing the bent piping closer to piping of the gas line GL1. Such a configuration heats the exhaust gas before being supplied to the reactors 4ab by heat exchange using the heat of the high-temperature gas (mixed gas) after passing through the reactors 4ab, and therefore enables effective use of heat.

Such a heat exchanger can be configured as a jacketed heat exchanger, immersed coil heat exchanger, a double tube heat exchanger, a shell and tube heat exchanger, a plate heat exchanger, a spiral heat exchanger, etc.

In the exhaust gas heating section 10, either the electric heater or the heat exchanger may be omitted.

The exhaust gas heating section 10 may have a combustion furnace or the like instead of the electric heater. However, when an electric heater is used, electric power (electrical energy) as a renewable energy source can be used to power it, which can reduce impact on the environment.

The renewable energy can be electrical energy from at least one of solar power, wind power, hydroelectric power, wave power, tidal power, biomass power, geothermal power, solar heat, and geothermal heat.

Upstream from the exhaust gas heating section 10 (e. g., a position between the gas-liquid separator and the guard reactor, the position being on the minor component removal section 7), a gas exhaust line may be branched off from the gas line GL1, and its end may be connected to a vent section disposed outside the gas production apparatus 1.

In this case, a valve may be provided on the exhaust gas line.

Even when the pressure in the gas production apparatus 1 (gas line GL1) increases beyond necessary, opening the valve can discharge (release) part of the exhaust gas from the vent section through the exhaust gas line. This can prevent the gas production apparatus 1 from being damaged by the increased pressure.

The gas line GL2 is connected to the reducing gas supply section 3 at one end. The gas line GL2 is connected to inlet ports of the reactors 41a and 41b of the reaction section 4 via the gas switching section 8 and the two respective gas lines GL3a and GL3b.

The reducing gas supply section 3 supplies reducing gas containing a reducing substance configured to reduce the reducing agent 4R oxidized by contacting carbon dioxide. The reducing gas supply section 3 according to the present embodiment is formed as a hydrogen generator configured to generate hydrogen by water electrolysis. This hydrogen generator is connected to a tank (reducing gas raw material storage section) 30 disposed outside the gas production apparatus 1 and configured to store water. Such a configuration allows the reducing gas containing hydrogen (reducing substance) supplied from the hydrogen generator (reducing gas supply section 3) to pass through the gas line GL2 and then to be supplied to the respective reactors 4ab.

The hydrogen generator can easily generate a large amount of hydrogen with a relatively low cost. Another advantage is that the condensed water generated in the gas production apparatus 1 can be reused. The electrical energy consumption in the hydrogen generator is large in the gas production apparatus 1, and therefore it is beneficial that the hydrogen generator uses electric power as a renewable energy source, as described above.

A byproduct hydrogen generator can also be used as a hydrogen generator. In this case, reducing gas containing byproduct hydrogen is supplied to each of the reactors 4ab. The byproduct hydrogen generator includes, for example, an apparatus configured to electrolyze sodium chloride solution, an apparatus configured to reform petroleum with steam, an apparatus configured to produce ammonia, and the like.

The gas line GL2 may be connected to a coke oven disposed outside the gas production apparatus 1 via a connection section, and exhaust gas from the coke oven may be used as reducing gas. In this case, the connection section functions as a reducing gas supply section. This is because the exhaust gas from the coke oven mainly contains hydrogen and methane, with the hydrogen at 50-60 vol%.

A reducing gas heating section 11 is disposed on the gas line GL2. This reducing gas heating section 11 is configured to heat the reducing gas before being supplied to the reactors 4ab. Pre-heating the reducing gas in the reducing gas heating section before reaction (before oxidation) allows further acceleration of the reduction (recovery) reaction by the reducing gas for the reducing agent 4R in the reactors 4ab.

The reducing gas heating section 11 can be configured similarly to the above-described exhaust gas heating section 10. The reducing gas heating section 11 may be an electric heater only, a heat exchanger only, or a combination of an electric heater and a heat exchanger, and may preferably be a heat exchanger only or a combination of an electric heater and a heat exchanger.

When the reducing gas heating section 11 has a heat exchanger, heat of high-temperature gas (e.g., mixed gas) after passing through the reactors 4ab can be used for heat exchange to heat the reducing gas before being supplied to the reactors 4ab, i.e., heat can be effectively used.

In such an above-described configuration, for example, switching the gas line (channel) in the gas switching section 8 can supply, via the gas line GL3a, the exhaust gas to the reactors 41a and 42a accommodating the reducing agent 4R before oxidation, and can supply, via the gas line GL3b, the reducing gas to the reactors 41b and 42b accommodating the reducing agent 4R after oxidation. In this stage, the reaction represented by following Equation 1 proceeds in the reactors 41a and 42a, and the reaction represented by following Equation 2 proceeds in the reactors 41b and 42b.

Following Equations 1 and 2 represent a case as an example where the reducing agent 4R contains iron oxide (FeOₓ₋₁).

Equation 1: CO₂+FeOₓ₋₁→CO+FeOₓ

Equation 2: H₂+FeOₓ→H₂O+FeOₓ₋₁

Thereafter, switching the gas line in the gas switching section 8 in the opposite direction to the above description can allow the reaction of above Equation 2 to proceed in the reactors 41a and 42a and can allow the reaction of above Equation 1 to proceed in the reactors 41b and 42b.

The reactions represented by Equations 1 and 2 above are both endothermic reactions. Therefore, the gas production apparatus 1 may further have a reducing agent heating section (not illustrated in FIG. 1) configured to heat the reducing agent 4R when the exhaust gas or reducing gas is brought into contact with the reducing agent 4R (i.e., when the exhaust gas or reducing gas reacts with the reducing agent 4R).

Such a reducing agent heating section can maintain the temperature in the reaction of the exhaust gas or reducing gas with the reducing agent 4R at a high temperature to suitably prevent or suppress reduction in the conversion efficiency from carbon dioxide into carbon monoxide and can further accelerate the recovery of the reducing agent 4R by the reducing gas.

However, depending on the reducing agent 4R, the reactions represented by Equations 1 and 2 above may be exothermic reactions. In this case, the gas production apparatus 1 may have a reducing agent cooling section configured to cool the reducing agent 4R instead of the reducing agent heating section. Such a reducing agent cooling section can suitably prevent degradation of the reducing agent 4R during the reaction of the exhaust gas or reducing gas with the reducing agent 4R and thereby suitably prevent or suppress reduction in the conversion efficiency from carbon dioxide into carbon monoxide and further accelerate the regeneration of the reducing agent 4R by the reducing gas.

In other words, the gas production apparatus 1 may have a reducing agent temperature regulation section configured to regulate the temperature of the reducing agent 4R depending on the type of the reducing agent 4R (exothermic or endothermic reaction).

The conversion rate from carbon dioxide into carbon monoxide in the reactors 4ab may be 80% or more, preferably be 82. 5% or more, 85% or more, 87. 5% or more, or 90% or more, more preferably be 92. 5% or more, and even more preferably be 95% or more. The upper limit of the conversion rate from carbon dioxide into carbon monoxide is usually about 98%.

Such a conversion rate can be set by adjusting the type of the reducing agent 4R used, the concentration of carbon dioxide in the exhaust gas, the type of the reducing substance, the concentration of the reducing substance in the reducing gas, the temperature in the reactors 4ab, the flow rates (velocities) of the exhaust gas and reducing gas supplied to the reactors 4ab, the timing of switching between the exhaust gas and the reducing gas, etc.

The conversion rate (%) from carbon dioxide into carbon monoxide is a value calculated by an expression: COₒᵤₜ/CO₂ᵢₙ×100. CO₂ᵢₙ represents an amount by mole of carbon dioxide supplied to the reactors 4ab, and COₒᵤₜ represents an amount by mole of carbon monoxide discharged from the reactors 4ab when carbon dioxide is converted into carbon monoxide by contact (reaction) with the reducing agent 4R.

The conversion rate from hydrogen into water in the reactors 4ab may be 40% or more, preferably be 45% or more, and more preferably be 50% or more. The upper limit of the conversion rate from hydrogen to water is usually about 85%.

Such a conversion rate can be set by adjusting the type of the reducing agent 4R used, the concentration of carbon dioxide in the exhaust gas, the type of the reducing substance, the concentration of the reducing substance in the reducing gas, the temperature in the reactors 4a and 4b, the flow rates (speeds) of the exhaust gas and reducing gas supplied to the reactors 4ab, the timing of switching between the exhaust gas and the reducing gas, etc.

The conversion rate of hydrogen into water (%) is a value calculated with an expression (H₂ᵢₙ-H₂ₒᵤt)/H₂ᵢₙ ×100. H₂ᵢₙ represents an amount by mole of hydrogen supplied to the reactors 4ab, and H₂ₒᵤₜ represents an amount by mole of hydrogen passing through the reactors 4ab in its unreacted state and discharged.

Gas lines GL4a and GL4b are respectively connected to outlet ports of the reactors 42a and 42b and are joined at the gas joint section J4, forming the gas lines GL4. Valves (not illustrated) may be disposed on the gas lines GL4a and GL4b, respectively, as needed.

For example, regulating the valve opening can adjust the passing speeds of the exhaust gas and reducing gas passing through the reactors 4ab (i.e., the treatment rate by the reducing agent 4R on the exhaust gas and the treatment rate by the reducing gas on the reducing agent 4R).

In the present embodiment, the reaction section 4 mainly has the reactors 4ab and the gas switching section 8.

At an end of the gas line GL4 opposite to the reactors 42a and 42b is connected a product gas discharge section 40 through which the product gas is discharged from the gas production apparatus 1.

A gas purification section 9 is further disposed on the gas line GL4.

The gas purification section 9 is configured to purify carbon monoxide from the mixed gas and thereby to recover product gas containing high concentrations of carbon monoxide. In a case where carbon monoxide concentration in the mixed gas is high enough, the gas purification section 9 may be omitted.

Such a gas purification section 9 may include, for example, at least one of treatment unit of a condenser, a gas-liquid separator, a gas separator, a separation membrane, and a scrubber (absorption tower).

In a case where two or more treatment units are used, the arrangement order thereof is not particularly limited, but when a condenser, a gas-liquid separator, and a gas separator are combined, they may be arranged in this order. This can further improve the efficiency of purifying carbon monoxide from the mixed gas.

The condenser is configured to cool the mixed gas. Thereby, condensed (liquid) water is produced.

Such a condenser may include a jacket-type condenser having a jacket for allowing coolant to pass around the pipes, a multitubular cooling system having a configuration similar to the reactors 4ab (see FIG. 2) and allowing mixed gas to pass through the tubes 41 and coolant to pass through a space 43 around the tubes 41, an air-fin cooler, and the like.

The gas-liquid separator is configured to separate the condensed water produced from the mixed gas during cooling the mixed gas in the condenser. The condensed water is beneficial to dissolving and removing unwanted gas components (especially carbon dioxide) remaining in the mixed gas.

The gas-liquid separator may have a configuration similar to the configuration of the gas-liquid separator in the minor component removal section 7 or may preferably be a simple container. In this case, at the gas-liquid interface inside the container, the container may have a filter configured to allow passage of gas but to block passage of liquid.

In this case, a liquid line may be connected to the bottom part of the container, and a valve may be disposed on the liquid line. With such a configuration, opening the valve allows the condensed water stored in the container to be discharged (released) from the gas production apparatus 1 via the liquid line.

In addition, a drain trap may be disposed downstream from the valve on the liquid line. This can allow, even in a case where the valve malfunctions and carbon monoxide or hydrogen leak into the liquid line, the leaking to be stored in the drain trap and prevented from being discharged from the gas production apparatus 1. Instead of this drain trap, or together with the drain trap, a valve malfunction detection function or redundancy against valve malfunctions may be provided.

The liquid line may be connected to the tank 30 described above so that the condensed water to be discharged is reused.

The gas separator may include, for example, one or more of a low-temperature separation (deep cooling) separator, a pressure swing adsorption (PSA) separator, a membrane separation separator, a temperature swing adsorption (TSA) separator, a separator using a porous coordination polymer (PCP), which is a complex of a metal ion (e.g., copper ion) and an organic coordination ligand (e.g., 5-azidoisophthalic acid), a separator using amine absorption, and the like.

A valve may be disposed between the gas-liquid separator and the gas separator on the gas line GL4. In this case, regulating the valve's opening degree can adjust the treatment rate on the mixed gas (production rate of product gas).

In this embodiment, the concentration of carbon monoxide in the mixed gas emitted from the gas-liquid separator is 75-100 vol% of the total mixed gas.

Therefore, in fields where the product gas can be used with carbon monoxide at relatively low concentrations (75-90 vol%), carbon monoxide can be supplied directly from the mixed gas to the next process without purification. In other words, the gas separator can be omitted.

Such fields include, for example, a field of synthesizing valuable carbon substances (e.g., ethanol, etc.) from the product gas by microorganism (e.g., Clostridium, etc.) fermentation, a field of producing steel by using the product gas as fuel or a reducing agent, a field of producing electrical devices, a field of synthesizing chemical products (e.g., phosgene, acetic acid, etc.) from carbon monoxide as a synthetic raw material, and the like.

On the other hand, in fields needing the product gas to contain carbon monoxide at relatively high concentrations (>90 vol%), the product gas containing carbon monoxide at high concentrations is obtained by purifying carbon monoxide from the mixed gas.

Such fields include, for example, a field of using the product gas as a reducing agent (blast furnace), a field of using the product gas as fuel to generate electricity by thermal power, a field of using the product gas as raw material to produce chemical products, a field of fuel cells using the product gas as fuel, and the like.

In the present embodiment, the reactor 41a and the reactor 42a are connected in series by the gas line GL5a, and the reactor 41b and the reactor 42b are connected in series by the gas line GL5b. A removal section 19 is disposed on each of the gas lines GL5a and GL5b.

The removal section 19 is configured to allow at least one of the following: separation between unreacted hydrogen (reducing substance) and water (product derived from the reducing gas) produced by contact (reaction) between the reducing agent 4R and hydrogen (reducing substance), and separation between unreacted carbon dioxide and carbon monoxide produced by contact between the reducing agent 4R and carbon dioxide.

That is, the removal section 19 is connected between the two of the reactors 4ab and includes a gas-liquid separating mechanism (phase separating mechanism) 19' configured to carry out gas-liquid separation between a gas component and a liquid component (separated component), which is produced by cooling, and thereby to remove the product derived from the gas supplied to the reactors 4ab.

Such a configuration can remove the product derived from the gas supplied to the reactors 41a and 41b, preventing reduction in the reaction efficiency in the reactors 42a and 42b, i.e., the conversion efficiency from carbon dioxide into carbon monoxide and the reduction (regeneration) efficiency for the reducing agent 4R in oxidized state. Hence, carbon monoxide (valuable carbon substance) can be efficiently produced from carbon dioxide.

For example, the produced water and carbon monoxide is discharged to the discharge lines 19a and 19b. Meanwhile, unreacted hydrogen and unreacted carbon dioxide are discharged to the gas lines GL5a and GL5b.

The gas discharged to the discharge lines 19a and 19b may contain water or other gas components other than carbon monoxide, and the gas discharged to the gas lines GL5a and GL5b may also contain hydrogen or other gas components other than carbon dioxide.

In the following, a description will be given of a gas-liquid separating mechanism 19' configured to carry out gas-liquid separation between hydrogen as the reducing substance and water as the product.

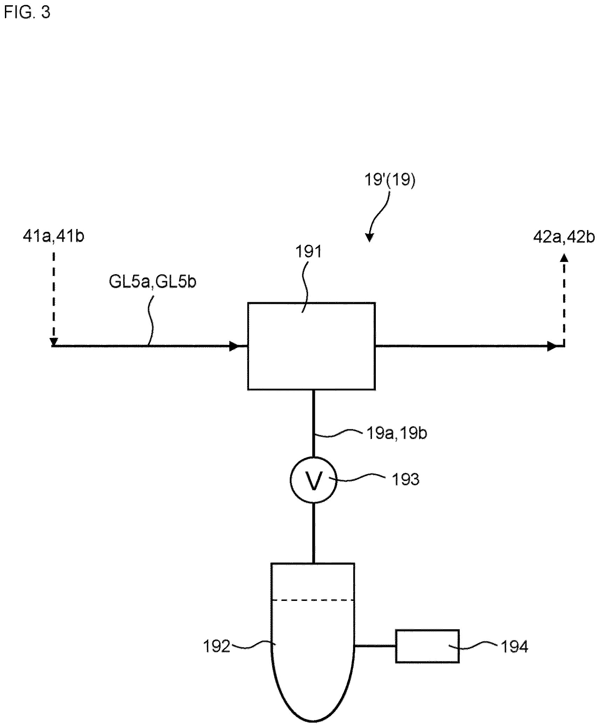

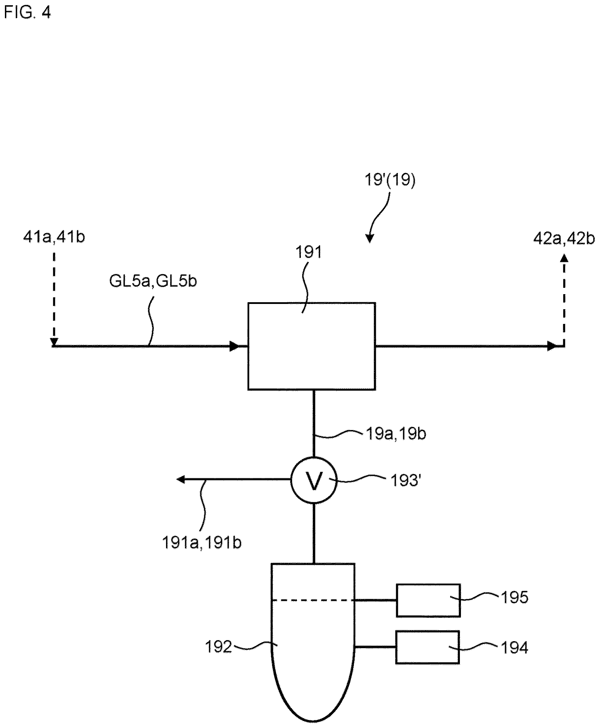

FIG. 3 is a schematic diagram illustrating a configuration of the gas-liquid separating mechanism. FIG. 4 is a schematic diagram illustrating another configuration of the gas-liquid separating mechanism.

The gas-liquid separating mechanism 19' illustrated in FIG. 3 has a condenser 191, a tank (storage section) 192, and a check valve 193.

The condenser 191 is disposed on each of the gas lines GL5a and GL5b. As this condenser 191, a condenser having a configuration similar to the above-described condenser of the gas purification section 9.

The condenser 191 is connected to the tank 192 configured to store water (liquid component) via each of the discharge lines 19a and 19b.

Such a gas-liquid separating mechanism 19' has a configuration such that the liquid component (separated component) does not flow into the reactors 4ab. This can further improve the conversion efficiency (conversion ratio) from carbon dioxide into carbon monoxide and the conversion efficiency (conversion ratio) from hydrogen into water.

Specifically, as illustrated in FIG. 3, the gas-liquid separating mechanism (phase separating mechanism) 19' is disposed at a position vertically lower than the reactors 4ab, and a check valve 193 is disposed between the gas-liquid separating mechanism 19' and the reactors 4ab. Such a relatively simple configuration can prevent the liquid component from flowing into the reactors 4ab.

Here, "a position vertically lower than the reactors 4ab" is not limited to a position strictly vertically lower than the reactors 4ab, but includes positions obliquely vertically lower than the reactors 4ab.

Only one configuration may be adopted from the configurations such that the gas-liquid separating mechanism 19' is disposed at a position vertically lower than the reactors 4ab, and such that the check valve 193 is disposed between the gas-liquid separating mechanism 19' and the reactors 4ab. This case can also prevent well the liquid component from flowing into the reactors 4ab.

A check valve 193 is disposed on each of the discharge lines 19a and 19b. This check valve 193 may be, for example, a duckbill valve, poppet check valve, lift check valve, swing check valve, ball check valve, wafer check valve, foot check valve, or the like.

When the boiling point of the liquid component stored in the tank 192 (temperature at which the separated component vaporizes) is defined as X [°C], the cooling temperature in the gas-liquid separation mechanism 19' (condenser 191) may be about X-120°C or more and X°C or less and absolute zero or more, preferably be about X-100°C or more and X-10°C or less, and more preferably be about X-80°C or more and X-20°C or less. Cooling the gas having passed through the reactors 4ab at such a cooling temperature can allow a substance, which is to be a liquid component when being cooled, to be condensed into a liquid state in a short time while avoiding freezing the substance.

In a case where the substance to be the liquid component is water, the cooling temperature may be about -20°C or more and 100°C or less, preferably be about 0°C or more and 90°C or less, and more preferably be about 20°C or more and 80°C or less.

The tank 192 has a pH meter (pH measurement section) 194 configured to measure pH of the stored water. The stored water may be made acidic by dissolution of carbon dioxide, hydrogen sulfide, etc. Providing the pH meter 194 allows monitoring of the pH of the water stored in the tank 192. In a case where the pH of the water accidentally becomes too low, a pH adjuster may be added to the water.

In consideration of such a case where the pH of the water reduces, the tank 192 configured to store water may be made of acid-resistant material. This can prevent or suppress corrosion of the tank 192 and extend the maintenance cycle of the gas production apparatus 1. The acid-resistant material includes, for example, various resin materials and metal materials such as stainless steel.

The gas-liquid separating mechanism 19' may be configured as illustrated in FIG. 4.

Hereinafter, a description will be given of the configuration illustrated in FIG. 4 with differences from the configuration illustrated in FIG. 3 focused on, and a description of similar matters will be omitted.

The gas-liquid separating mechanism 19' illustrated in FIG. 4 has a switching valve (channel blocking mechanism) 193' on each of the discharge lines 19a and 19b. In a case where the amount of the water stored in the tank 192 becomes greater than a predetermined amount, switching the switching valve 193' can allow the excess amount of the water to be discharged to each of the switching lines 191a and 191b.

In the gas-liquid separating mechanism 19' illustrated in FIG. 4, the tank 192 is connected to a liquid level sensor (liquid level detection section) 195 configured to detect the liquid level position of the water (liquid component).

Providing the liquid level sensor 195 allows the switching valve 193' to block the discharge line 19a or 19b, which are channels for transferring water (liquid component) to the tank (storage section) 192, depending on the liquid level position detected by the liquid level sensor 195, and to switch to the switching line 191a or 191b to discharge the water.

In this case, water may be returned to the tank 30.

The gas-liquid separation mechanism 19' having such a configuration can prevent the separated and removed water (liquid component) from flowing into the gas production apparatus 1 again, enabling stable operation of the gas production apparatus 1.

The water stored in the tank 192 may bubble or foam, and therefore the liquid level sensor 195 may have such a detection system that liquid level position detection is unaffected or not easily affected by the bubbling (foaming). Such a liquid level sensor 195 allows accurate determination of the amount of the water stored in the tank 192.

The liquid level sensor 195 employing such a detection system may include a differential pressure liquid level gauge, an impedance liquid level gauge, or a capacitance liquid level gauge.

In a case where the water bubbles or foams, a defoaming agent (e.g., surfactant) may be added to the water, or gas may be blown to the water.

The removal section 19 may have a separation membrane instead of or in addition to the gas-liquid separating mechanism 19'. In a case where the gas-liquid separating mechanism 19' is combined with a separation membrane, the separation membrane may be disposed upstream, downstream, or both upstream and downstream from the gas-liquid separating mechanism 19'.

The separation membrane either allows passage of product molecules and blocks passage of all other molecules, or blocks passage of product molecules and allows passage of all other molecules.

Such a separation membrane may be made of metals, inorganic oxides, or metal organic frameworks (MOFs).

The metals include, for example, titanium, aluminum, copper, nickel, chromium, cobalt, alloys containing these metals, and the like. When being made of metal, the separation membrane may be porous with a porosity of 80% or more.

The inorganic oxides include, for example, silica, zeolite, and the like.

The metal-organic frameworks include, for example, a framework formed by zinc nitrate hydrate and terephthalic acid dianion, a framework formed by copper nitrate hydrate and trimesic acid trianion, and the like.

The separation membrane may be made of a porous material having continuous pores (pores penetrating a cylinder wall), which are adjacent pores connected to each other. A separation membrane with such a configuration can increase the permeability of water or carbon monoxide, and thereby allow smoother and more reliable separation between water and hydrogen and/or separation between carbon monoxide and carbon dioxide.

The porosity of the separation membrane is not particularly limited, but may be 10-90%, and preferably be 20-60%. This allows the separation membrane to maintain a high permeability to water or carbon monoxide while preventing excessive reduction in the mechanical strength of the membrane.

The shape of the separation membrane is not particularly limited, and may be cylindrical shape, polygonal cylindrical shape such as square cylindrical shape and hexagonal cylindrical shape, or the like.

From the viewpoint of more efficient conversion from carbon dioxide into carbon monoxide, it is effective to increase the reduction (regeneration) efficiency for the reducing agent 4R in oxidized state.

In this case, the average pore size of the separation membrane may be 600 pm or less, and preferably be 400-500 pm. This allows more efficient separation between water and hydrogen.

The separation membrane is usually used in a state of being accommodated in a housing. In this case, the space outside the separation membrane in the housing may be depressurized or may allow a carrier gas (sweep gas) to pass therethrough. The carrier gases includes, for example, inert gases such as helium and argon, and the like.

The separation membrane may be hydrophilic. Such a hydrophilic separation membrane increases the affinity of water for the separation membrane, allowing water to penetrate the separation membrane more smoothly.

Methods for imparting hydrophilicity to the separation membrane include a method of increasing the polarity of the separation membrane by changing the proportion of a metal element in the inorganic oxide (e.g., increasing Al/Si ratio), a method of coating the separation membrane with a hydrophilic polymer, a method of treating the separation membrane with a coupling agent having a hydrophilic group (polar group), a method of providing plasma treatment or corona discharge treatment on the separation membrane, and the like.

The surface potential of the separation membrane may be regulated for controlling its affinity for water.

In a case where separation between carbon monoxide and carbon dioxide is prioritized on the separation membrane, or in a case where both separation between water and hydrogen and separation between carbon monoxide and carbon dioxide are to be performed simultaneously on the separation membrane, the constituent material, porosity, average pore size, degree of hydrophilicity or hydrophobicity, surface potential, etc. of the separation membrane may be appropriately combined.

The tubes 41 of the reactors 4ab could be made of a separation membrane, but in this case, the temperature (reaction temperature) of the reactors 4ab cannot be set to a high temperature because heat may impair the separation membrane.

In contrast, disposing the separation membrane outside the reactors 4ab enables the temperature of the reactors 4ab to be set to a relatively high temperature, which increases the conversion efficiency from carbon dioxide into carbon monoxide and regeneration (reduction) efficiency by the reducing gas for the reducing agent 4R.

Next, the method for using (action) of the gas production system 100 will be explained.
[1] First, switching the gas line (channel) in the gas switching section 8 allows the connection section 2 and the reactor 41a to communicate and the reducing gas supply section 3 and the reactor 41b to communicate.
[2] Next, in this state, the furnace 20 starts to supply the exhaust gas via the connection section 2.
[3] Subsequently, the exhaust gas passes through the oxygen removal apparatus (concentration adjustment section 5). This removes oxygen from the exhaust gas and increases the concentration of carbon dioxide in the exhaust gas.
[4] Next, the exhaust gas passes through the compression section 6. This increases the pressure of the exhaust gas.
[5] Thereafter, the exhaust gas passes through the minor component removal section 7. This removes, from the exhaust gas, the condensed water and the inactivation component, the condensed water being produced when the exhaust gas is condensed in the compression section 6, the inactivation component reducing the activity of the reducing agent 4R.
[6] Next, the exhaust gas passes through the exhaust gas heating section 10. This heats the exhaust gas.
[7] Then, the exhaust gas is supplied to the reactor 41a. In the reactor 41a, the reducing agent 4R reduces carbon dioxide in the exhaust gas into carbon monoxide. This contact with carbon dioxide makes the reducing agent 4R oxidized state.

The temperature (reaction temperature) in the reactor 41a (temperature of the exhaust gas and the reducing agent 4R) in the above step [7] may be 500°C or more, preferably be 600°C or more, more preferably be 650-1100°C, and even more preferably be 700-1000°C. Setting the reaction temperature to the above range, for example, prevents or hinders the endothermic reaction of converting carbon dioxide into carbon monoxide from rapidly lowering temperature of the reducing agent 4R, enabling smoother proceeding of the reduction reaction for carbon dioxide in the reactor 41a.

[9] The exhaust gas is discharged from the reactor 41a into the gas line GL5a.

Thereafter, carbon monoxide may be removed from the exhaust gas when the exhaust gas passes through the removal section 19, and may then be supplied to the gas line GL4 via the discharge line 19a.

[10] Next, the exhaust gas is supplied to the reactor 42a. Also in the reactor 42a, the reducing agent 4R reduces carbon dioxide in the exhaust gas to carbon monoxide. At this time, contact with carbon dioxide makes the reducing agent 4R oxidized state.

The condition in the reactor 42a may be set similarly to the condition in the reactor 41a.

[11] In parallel with the above steps [2] to [10], water (reducing gas raw material) is supplied from the tank 30 to the hydrogen generator (reducing gas supply section 3) and hydrogen is produced from the water.

[12] Next, the reducing gas containing hydrogen passes through the reducing gas heating section 11. This heats the reducing gas.

[13] Subsequently, the reducing gas is supplied to the reactor 41b. In the reactor 41b, contact with the reducing gas (hydrogen) reduces (regenerates) the reducing agent 4R in the oxidized state. At this time, water is produced.

The temperature (reaction temperature) in the reactor 41b (temperature of the reducing gas and the reducing agent 4R) in the above step [13] may be 500°C or more, preferably be 600°C or more, more preferably be 650-1100°C, and even more preferably be 700-1000°C. Setting the reaction temperature to the above range, for example, prevents or hinders endothermic reaction during the reduction (regeneration) of the reducing agent 4R in oxidized state from rapidly lowering temperature of the reducing agent 4R, enabling smoother proceeding of the reduction reaction for the reducing agent 4R in the reactor 41b.

[14] The reducing gas is discharged from the reactor 41b into the gas line GL5b.

The water is then removed from the reducing gas as the reducing gas passes through the removal section 19 and is discharged via the discharge line 19b. This reduces the amount of water contained in the mixed gas.

[15] Thereafter, the reducing gas is supplied to the reactor 42b. In the reactor 42b, contact with the reducing gas (hydrogen) reduces (regenerates) the reducing agent 4R in oxidized state. At this time, water is produced.

The condition in the reactor 42b may be set similarly to the condition in the reactor 41b.

[16] Next, the gases having passed through the reactors 42a and 42b are joined, which produces mixed gas. At this point, the temperature of the mixed gas is typically 600-650°C. At this point, the temperature of the mixed gas being in the above range indicates (or represents) that the temperature in the reactors 4ab is maintained at a high enough temperature, suggesting efficient proceeding of the conversion by the reducing agent 4R from carbon dioxide into carbon monoxide and the reduction by the reducing gas for the reducing agent 4R.

[17] Next, the mixed gas is cooled to 100-300°C before reaching the gas purification section 9.

[18] Subsequently, the mixed gas passes through the gas purification section 9. This removes, for example, the generated condensed water, carbon dioxide dissolved in the condensed water, etc. As a result, carbon monoxide is purified from the mixed gas and product gas containing high concentrations of carbon monoxide is obtained.

The temperature of the product gas obtained is 20-50°C.

[19] Next, the product gas is discharged from the gas production apparatus 1 via the product gas discharge section 40 and is subjected to the next step.

### SECOND EMBODIMENT

The reaction section 4 may also be configured as described below.

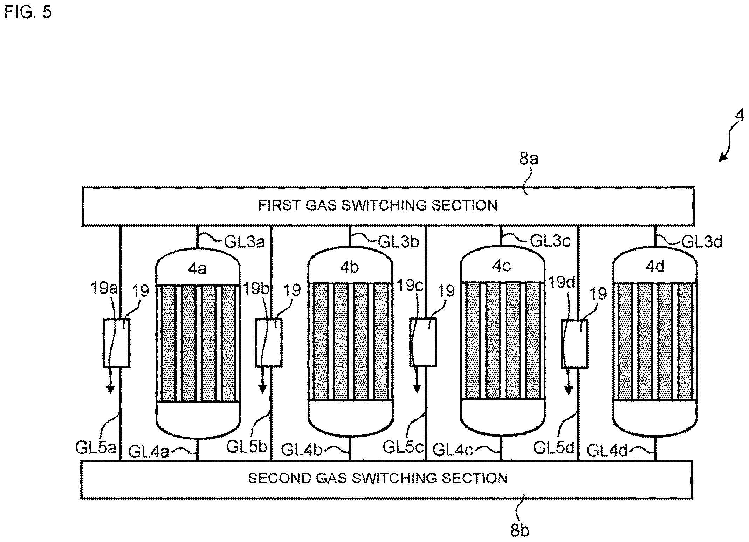

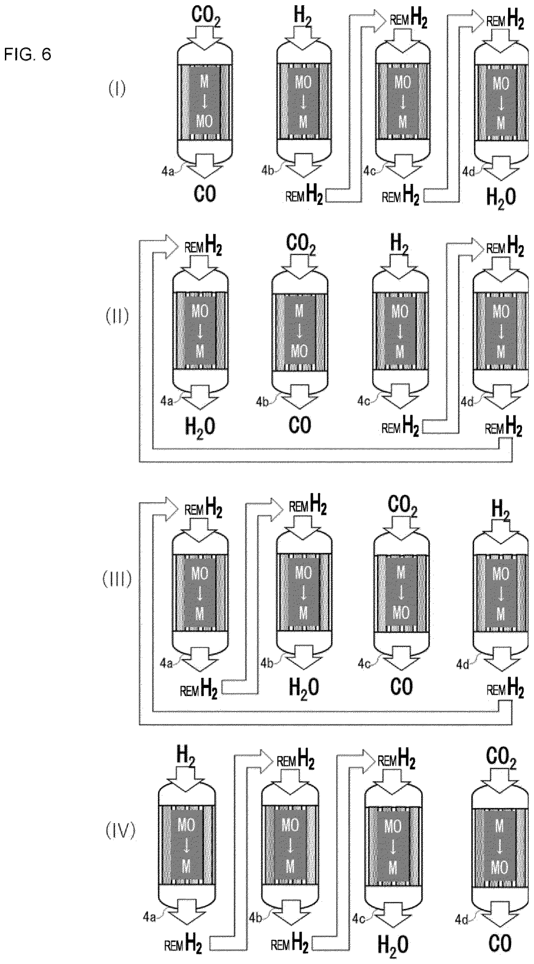

FIG. 5 is a schematic diagram illustrating a configuration of a reaction section according to the second embodiment. FIG. 6 is a schematic diagram illustrating a method for switching gas to pass through the reactor according to the second embodiment.

Hereinafter, a description will be given of the reaction section 4 according to the second embodiment with the differences from the reaction section 4 according to the first embodiment focused on, and a description of similar matters will be omitted.

The reaction section 4 according to the second embodiment has a first gas switching section 8a, four reactors 4a to 4d, and a second gas switching section 8b.

The first gas switching section 8a is connected to the inlet ports of the reactors 4a to 4d respectively via the gas lines GL3a to GL3d.

Gas lines GL4a through GL4d are respectively connected to outlet ports of the reactors 4a to 4d, join at the second gas switching section 8b, and then form a gas line GL4.

Four gas lines GL5a to GL5d connects the first gas switching section 8a and the second gas switching section 8b.

A removal section 19 is disposed on each of the gas lines GL4a to GL4d.
With such a configuration, switching the gas line (channel) at the first gas switching section 8a and the second gas switching section 8b, for example, supplies the exhaust gas (oxidizing gas) to and allows it to pass through one reactor of the reactors 4a to 4d, while supplying the reducing gas and allowing it to pass through the remaining three reactors of the reactors 4a to 4d consecutively in this order.

In other words, in the present embodiment, three reactors are connected in series, and the removal section 19 configured to remove a product derived from the gas supplied to the reactors is connected between adjacent two reactors.

In the present embodiment, of the plurality of reactors 4a to 4d, one reactor supplied with the exhaust gas functions as a first reactor, and three reactors consecutively supplied with the reducing gas when the exhaust gas is supplied to the first reactor function as the second reactor.

Specifically, in the first turn represented as (I) in FIG. 6, exhaust gas (carbon dioxide) is supplied via the gas line GL3a to the reactor (first reactor) 4a, and the exhaust gas (carbon monoxide) having passed through the reactor 4a is discharged through the gas line GL4a.

Meanwhile, with respect to the remaining reactors 4b to 4d, reducing gas (hydrogen) is first supplied via the gas line GL3b to the reactor (firstly positioned (headmost) second reactor) 4b, and subsequently, the reducing gas (remaining hydrogen (REM H₂ in the drawings)) having passed through the reactor 4b is supplied via the gas line GL4b, gas line GL5c, and gas line GL3c to the reactor (secondarily positioned second reactor) 4c. Water is removed by the removal section 19 on the gas line GL5c and discharged via the discharge line 19c.

Thereafter, the reducing gas (residual hydrogen) having passed through the reactor 4c can be supplied via the gas line GL4c, gas line GL5d, and gas line GL3d to the reactor (third positioned second reactor) 4d, and the reducing gas (water) having passed through the reactor 4d can be discharged via the gas line GL4d. Water is removed by the removal section 19 on the gas line GL5d and discharged via the discharge line 19d.

Next, in the second turn represented as (II) in FIG. 6, the exhaust gas is supplied to and allowed to pass through the reactor (first reactor) 4b, while reducing gas is supplied to and allowed to pass through the reactors (second reactor) 4c, 4d, and 4a consecutively this order.

Then, in the third turn represented by (III) in FIG. 6, exhaust gas is supplied to and allowed to pass through the reactor (first reactor) 4c, while reducing gas is supplied to and allowed to pass through the reactors (second reactor) 4d, 4a, and 4b consecutively in this order.

Next, in the fourth turn represented by (IV) in FIG. 6, exhaust gas is supplied to and allowed to pass through the reactor (first reactor) 4d, while reducing gas is supplied to and allowed to pass through the reactors (second reactor) 4a, 4b, and 4c consecutively in this order.

In the present embodiment, repeating a plurality cycles, with the series of operations from the first turn to the fourth turn as one cycle, enables continuous and stable conversion from carbon dioxide into carbon monoxide.

For example, in a case of using a reducing agent 4R with which reduction efficiency by hydrogen (reducing substance) for the reducing agent 4R in oxidized state is lower than conversion efficiency from carbon dioxide into carbon monoxide, passing only once of reducing gas through only one reactor would waste hydrogen (remaining hydrogen) having not been fully used in the reduction for the reducing agent 4R in oxidized state. On the other hand, the present embodiment allows the reducing gas to consecutively pass through three reactors, that is, three times through one reactor. This prevents hydrogen (reducing gas) from being wasted.

Using three or more reactors can provide a reactor not being passed through by the exhaust gas and reducing gas. This enables, during maintenance of normal operation to produce the product gas (carbon monoxide), another operation on a reactor not being used for the normal operation.

For example, during the conversion from carbon dioxide into carbon monoxide (valuable carbon substance), carbon may be deposited on the surface of the reducing agent 4R, which reduces the conversion efficiency. In this case, operation of supplying oxygen to the reactor not being used for the normal operation can remove the carbon deposited on the surface of the reducing agent 4R by combustion and thereby regenerates the reducing agent 4R.

In this case, inert gas (e.g., nitrogen gas) may be introduced into the reactor to purge it before and after supplying oxygen to the reactor. This prevents unwanted contact between oxygen and the reducing gas, preventing their explosive reaction.

Such an above-described gas production apparatus 1 (gas production system 100) allows efficient production of the valuable carbon substance by using oxidizing gas containing carbon dioxide and reducing gas containing reducing substance.

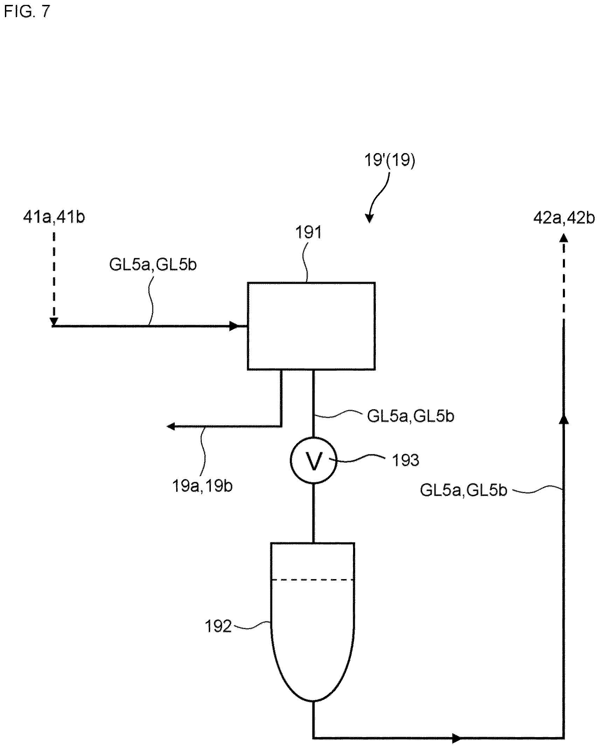

The gas-liquid separating mechanism 19' configured to carry out gas-liquid separation between carbon dioxide and carbon monoxide as a product may be configured as illustrated in FIG. 7.

FIG. 7 is a schematic view illustrating another configuration of the gas-liquid separating mechanism.

A description will be given of the configuration illustrated in FIG. 7 with the differences from the configuration illustrated in FIG. 3 focused on, and a description of similar matters will be omitted.

The gas-liquid separating mechanism 19' illustrated in FIG. 7 has a condenser 191, a check valve 193, and a tank 192 on gas lines GL5a and GL5b, in order from the upstream side. The pH meter 194 is omitted. In this configuration example, the condenser 191 and the tank 192 are connected to a pressurizer configured to pressurize the inner side of the condenser 191 and the tank 192 so that carbon dioxide is recovered as liquid.

The tank 192 stores the carbon dioxide as a liquid component resulting from the cooling and the pressurization in the condenser 191. Carbon monoxide as a gas component is discharged from the discharge lines 19a and 19b. The discharge lines 19a and 19b may be connected to the gas line GL4.

In the configuration illustrated in FIG. 7, the carbon dioxide stored in the tank 192 is vaporized again and then supplied to the reactors 42a and 42b. Hence, a vaporizer may be disposed at a position on the gas line GL5a and downstream from the tank 192.

When the pressurizer is omitted, carbon dioxide may be sublimated and recovered as a solid component (separated component). In this case, the phase separating mechanism functions as a gas-solid separating mechanism configured to carry out gas-solid separation between the solid component and the gas component. In this case, the temperature X [°C] at which the separated component vaporizes corresponds to the sublimation point of the solid component (carbon dioxide in solid state).

The phase separating mechanism may be configured to carry out phase separation between the gas component and a separated component that includes both liquid component and solid component. It is to be noted that the gas-liquid separating mechanism configured to carry out gas-liquid separation between the liquid component and the gas component is advantageous from the viewpoint that it can broaden the range of apparatus configuration choices without complicating the apparatus configuration.

In addition, the present disclosure may be provided in each of the following aspects.
(1) A gas production apparatus comprising: at least two reactors connected in series and configured to supply oxidizing gas containing carbon dioxide and reducing gas containing reducing substance, each reactor accommodating at least one of metal and metal oxide, the least one of metal and metal oxide being configured to produce a valuable carbon substance by reducing the carbon dioxide or accelerating reaction of the carbon dioxide with the reducing substance; and a removal section connected between the two reactors and having: a phase separating mechanism configured to carry out phase separation between a separated component and a gas component, and thereby to remove a product derived from gas supplied to the reactors, the separated component containing at least one of a liquid component and a solid component, which are produced by cooling.
(2) The gas production apparatus according to (1), wherein the temperature in the reactors is equal to or more than 500°C.
(3) The gas production apparatus according to (1) or (2), wherein the gas production apparatus is configured to prevent the separated component from flowing into the reactors.
(4) The gas production apparatus according to (3), having at least one of: a configuration of disposing the phase separating mechanism at a position vertically lower than the reactors; and a configuration of disposing a check valve between the phase separating mechanism and the reactors.
(5) The gas production apparatus according to any one of (1) to (4), wherein when the temperature at which the separated component vaporizes is defined as X [°C], a temperature of the cooling in the phase separating mechanism is X-120°C or more and X°C or less and is absolute zero or more.
(6) The gas production apparatus according to any one of (1) to (5), wherein the phase separating mechanism is a gas-liquid separating mechanism configured to perform gas-liquid separation between the liquid component and the gas component.
(7) The gas production apparatus according to (6), further comprising: a storage section configured to store the liquid component; a liquid level detection section configured to detect a liquid level position of the liquid component; and a channel blocking mechanism configured to block a channel for transferring the liquid component to the storage section depending on the liquid level position detected by the liquid level detection section.
(8) The gas production apparatus according to (7), wherein the liquid level detection section includes a differential pressure liquid level gauge, an impedance liquid level gauge, or a capacitance liquid level gauge.
(9) The gas production apparatus according to any one of (1) to (8), wherein: the at least one of metal and metal oxide functions as a reducing agent for reducing the carbon dioxide, and the reducing agent is brought into an oxidized state by contact with the carbon dioxide and is reduced by contact with the reducing substance.
(10) The gas production apparatus according to (9), wherein each of the reactors is configured to switch gas to be supplied between the oxidizing gas and the reducing gas.
(11) The gas production apparatus according to (9) or (10), wherein the product is derived from the reducing gas.
(12) The gas production apparatus according to any one of (9) to (11), wherein the product is produced by reaction of the reducing substance with the at least one of metal and metal oxide.
(13) The gas production apparatus according to any one of (1) to (12), wherein the reducing substance is hydrogen, and the product is water.
(14) The gas production apparatus according to (13), further comprising a pH measurement section configured to measure a pH of the water.
(15) The gas production apparatus according to (13) or (14), further comprising a storage section having a tank configured to store the water, the tank being made of an acid resistant material.

Of course, the present disclosure is not limited to those.

Various embodiments of the present disclosure have been described, but as described, these are presented as examples and are not intended to limit the scope of the disclosure. Novel embodiments can be implemented in various other forms, and various omissions, replacements, and modifications can be made within the scope of the spirit of the disclosure. The embodiments and its modifications are included in the scope and the spirit of the disclosure and are included in the scope of the invention described in claims and the equivalent scope thereof.

For example, as compared to the above embodiments, the gas production apparatus according to the present disclosure may have any other additional configuration, may have a configuration replaced with any configuration configured to exhibit the similar function, and may be configured with part of configuration omitted.

In the first and second embodiments above, a description is given of an example where at least one of metal and metal oxide functions as a reducing agent configured to reduce carbon dioxide, but the at least one of metal and metal oxide may function as a catalyst for accelerating reaction of carbon dioxide with a reducing substance. In this case, the reactor contains the catalyst and exhaust gas and reducing gas are supplied simultaneously into this reactor so that reverse water-gas shift reaction is carried out.

In the above first and second embodiments, a description is given of an example where reducing gas is gas containing hydrogen, but the reducing gas may be gas containing one selected from hydrocarbons (e.g., methane, ethane, acetylene, etc.) and ammonia, instead of or together with hydrogen.

In the above first embodiment, the number of reactors connected in series may be three or more, and in the above second embodiment, the number of reactors in series used as the second reactor (reactors on the reducing side for reducing the reducing agent 4R) may be two or may be four or more.

### [Description of Signs]

- 1:: gas production apparatus
- 2:: connection section
- 3:: reducing gas supply section
- 4:: reaction section
- 41:: tube
- 42:: housing
- 43:: space
- 4R:: reducing agent
- 4a:: reactor
- 4b:: reactor
- 4c:: reactor
- 4d:: reactor
- 41a:: reactor
- 41b:: reactor
- 42a:: reactor
- 42b:: reactor
- 5:: concentration adjustment section
- 6:: compression section
- 7:: minor component removal section
- 8:: gas switching section
- 8a:: first gas switching section
- 8b:: second gas switching section
- 9:: gas purification section
- 10:: exhaust gas heating section
- 11:: reducing gas heating section
- 19:: removal section
- 19':: gas-liquid separating mechanism
- 191:: condenser
- 192:: tank
- 193:: check valve
- 193':: switching valve
- 194:: pH meter
- 195:: liquid level sensor
- 20:: furnace
- 30:: tank
- 40:: product gas discharge section
- 100:: gas production system
- GL1:: gas line
- GL2:: gas line
- GL3a:: gas line
- GL3b:: gas line
- GL3c:: gas line
- GL3d:: gas line
- GL4:: gas line
- GL4a:: gas line
- GL4b:: gas line
- GL4c:: gas line
- GL4d:: gas line
- GL5a:: gas line
- GL5b:: gas line
- GL5c:: gas line
- GL5d:: gas line
- 19a:: discharge line
- 19b:: discharge line
- 19c:: discharge line
- 19d:: discharge line
- 191a:: switching line
- 191b:: switching line
- J4: gas: joint section

## Claims

1. A gas production apparatus comprising:
at least two reactors connected in series and configured to supply:
oxidizing gas containing carbon dioxide; and
reducing gas containing reducing substance,
each reactor accommodating at least one of metal and metal oxide,
the least one of metal and metal oxide being configured to produce a valuable carbon substance by reducing the carbon dioxide or accelerating reaction of the carbon dioxide with the reducing substance; and
a removal section connected between the two reactors and having:
a phase separating mechanism configured to carry out phase separation between a separated component and a gas component, and thereby to remove a product derived from gas supplied to the reactors, the separated component containing at least one of a liquid component and a solid component, which are produced by cooling.

2. The gas production apparatus according to claim 1, wherein
the temperature in the reactors is equal to or more than 500°C.

3. The gas production apparatus according to claim 1 or 2, wherein
the gas production apparatus is configured to prevent the separated component from flowing into the reactors.

4. The gas production apparatus according to claim 3, having at least one of:
a configuration of disposing the phase separating mechanism at a position vertically lower than the reactors; and
a configuration of disposing a check valve between the phase separating mechanism and the reactors.

5. The gas production apparatus according to any one of claims 1 to 4, wherein
when the temperature at which the separated component vaporizes is defined as X [°C], a temperature of the cooling in the phase separating mechanism is X-120°C or more and X°C or less and is absolute zero or more.

6. The gas production apparatus according to any one of claims 1 to 5, wherein
the phase separating mechanism is a gas-liquid separating mechanism configured to carry out gas-liquid separation between the liquid component and the gas component.

7. The gas production apparatus according to claim 6, further comprising:
a storage section configured to store the liquid component;
a liquid level detection section configured to detect a liquid level position of the liquid component; and
a channel blocking mechanism configured to block a channel for transferring the liquid component to the storage section depending on the liquid level position detected by the liquid level detection section.

8. The gas production apparatus according to claim 7, wherein
the liquid level detection section includes a differential pressure liquid level gauge, an impedance liquid level gauge, or a capacitance liquid level gauge.

9. The gas production apparatus according to any one of claims 1 to 8, wherein:
the at least one of metal and metal oxide functions as a reducing agent for reducing the carbon dioxide, and
the reducing agent is brought into oxidized state by contact with the carbon dioxide and is reduced by contact with the reducing substance.

10. The gas production apparatus according to claim 9, wherein
each of the reactors is configured to switch gas to be supplied between the oxidizing gas and the reducing gas.

11. The gas production apparatus according to claim 9 or 10, wherein
the product is derived from the reducing gas.

12. The gas production apparatus according to any one of claims 9 to 11, wherein
the product is produced by reaction of the reducing substance with the at least one of metal and metal oxide.

13. The gas production apparatus according to any one of claims 1 to 12, wherein
the reducing substance is hydrogen, and the product is water.

14. The gas production apparatus according to claim 13, further comprising a pH measurement section configured to measure a pH of the water.

15. The gas production apparatus according to claim 13 or 14, further comprising a storage section having a tank configured to store the water, the tank being made of an acid resistant material.
